# EUROPEAN PATENT SPECIFICATION

(11) **EP 4 279 112 B1**
(45) Date of publication and mention of the grant of the patent: **07.10.2026**
(21) Application number: 22790793.8
(22) Date of filing: 25.03.2022
(51) Int. Cl.: A61M 27/00, A61F 2/90, A61F 2/852, A61F 2/91

(54) **URETERAL STENT AND MANUFACTURING METHOD THEREOF**
HARNRÖHRENSTENT UND DESSEN HERSTELLUNGSVERFAHREN
ENDOPROTHÈSE URÉTÉRALE ET SON PROCÉDÉ DE FABRICATION

(30) Priority: 19.04.2021 CN 202110418367
(43) Date of publication of application: 22.11.2023
(73) Proprietor: HONEST MEDICAL CHINA CO., LTD., Zhuhai, Guangdong 519031 (CN)
(72) Inventor: GUO, Aijun, Zhuhai, Guangdong 519031 (CN); LI, Zhirong, Zhuhai, Guangdong 519031 (CN); WANG, Jianjin, Zhuhai, Guangdong 519031 (CN)
(74) Representative: Zaboliene, Reda
(86) International application number: PCT/CN2022/082939
(87) International publication number: WO 2022/222684

(56) References cited:
- EP-B1- 1 478 414
- WO-A1-2020/110051
- CN-A- 101 496 754
- CN-A- 106 691 646
- CN-A- 109 906 100
- CN-A- 111 001 045
- CN-A- 112 107 782
- CN-A- 112 641 544
- CN-A- 113 198 083
- CN-U- 204 446 191
- CN-U- 209 220 581
- US-A1- 2004 138 644
- US-A1- 2014 180 392
- US-A1- 2016 256 296
- US-A1- 2017 156 842
- US-A1- 2019 083 766
- US-B1- 6 395 021
- US-B2- 6 887 215

## Description

### TECHNICAL FIELD

The disclosure relates to the technical field of medical instruments, and more particularly to a ureteral stent and a manufacturing method thereof.

### BACKGROUND

Invasive surgeries are applied to the treatment of ureteral obstruction, hydronephrosis and the like for diseases such as tumors, retroperitoneal fibrosis, and ureterostenosis. During surgeries, the ureteral stent for supporting the ureter may be placed within the ureter, to avoid ureterostenosis, ureter adhesions and ureteral obstruction, and enable the urine to flow from the renal pelvis to the bladder.

Conventional ureteral stents, which are widely used in hospitals and are referred to as pigtail stents, are usually made from polyurethane. They have high radial strength and are not readily deformable. Furthermore, a pigtail stent usually has a side wall provided with a number of through holes to facilitate better fluid guiding performance. However, the through holes may be blocked by proliferative tissues after prolonged periods of use, which may lead to stent failure in fluid guiding.

CN204446191 discloses a medical stent of a composite structure, which includes an outer layer, a middle layer of a mesh structure woven by fine metal wires, and an inner layer made of a film with a smooth surface. In order to reduce the adhesion of bacteria and proteins, the inner layer is preferably designed to be an ultra-thin PTFE or a HDPE. The outer layer is a polymer material used in traditional stents, and the optional materials of the outer layer include silicone rubber, polyurethane, polyethylene, nylon or modified nylon.

US 2016/256296A discloses an endoprosthesis, including both one or more wires forming a framework and covering layer covering the framework. The covering layer may cover the framework such that the one or more wires may be disposed completely around covering layer.

EP 1478414B discloses an intraluminal stent. The coated metallic filament comprises a metallic filament that is coated with two biodegradable polymeric material coating layers, inner coating layer and outer coating layer. Uncoated filaments may be knitted, woven, braided, coiled or otherwise shaped into a metallic reinforcing structure, which is subsequently coated with a biodegradable polymeric material.

### SUMMARY

The disclosure is to overcome at least an existing technical problem of the prior art. To this end, the disclosure provides a ureteral stent for effectively reducing ureteral stent failure in fluid guiding.

A ureteral stent according to a first aspect of the disclosure comprises: an inner structure, and an outer structure sleeved on the inner structure, wherein the outer structure is a tube-shaped structure that is radially compressible, and the outer structure is knitted with a first wire, the inner structure is a rope-shaped structure that is radially uncompressible, and the inner structure is woven with a second wire, and the second wire that is woven defines fluid guiding gaps for fluid guiding therein.

In some embodiments of the disclosure, the first wire may be an elastic thread.

In some embodiments of the disclosure, the first wire may be a memory alloy thread.

In some embodiments of the disclosure, the first wire may be bent to form loops passing through one another to constitute the outer structure.

In some embodiments of the disclosure, segments of the first wire interlacing in a first direction and a second direction may constitute the outer structure, wherein the first direction and the second direction may be perpendicular to each other.

In some embodiments of the disclosure, the inner structure may be at least made of a strand of second wire, and the knitted second wire may be provided with fluid guiding gaps for fluid guiding therein.

In some embodiments of the disclosure, both ends of the ureteral stent may be bent, and the two ends of the ureteral stent may be bent in opposite directions.

A manufacturing method of a ureteral stent according to a second aspect of the disclosure, for manufacturing the above ureteral stent, comprises steps as follows: preparing the outer structure with the first wire, and then sleeving the outer structure on the inner structure.

In some embodiments of the disclosure, using knitting method, in which the first wire is bent to form loops passing through one another to constitute the outer structure.

In some embodiments of the disclosure, using woven method, in which segments of the first wire interlace with one another in a first direction and a second direction to constitute the outer structure, wherein the first direction and the second direction are perpendicular to each other.

The ureteral stent of the disclosure can be applied as follows. During use, the tube-shaped outer layer may be radially compressed and then placed in the urethra by an implant device, to provide a support in the urethra. When the outer structure deforms, the outer structure supported by the inner structure would not deform too much to completely fold up due to the inner structure that provides a support inside the outer structure. In such a case, the urine can flow along the gaps formed by the first wire of the outer layer. Thus, the outer structure still has an effect on urinary catheterization. Consequently, the ureteral stent failure in fluid guiding can be effectively reduced.

Some additional aspects and advantages of the disclosure will be set forth in the description below, and some will be apparent from the description, or may be learned by practice of the invention. The ureteral stent of the present invention is defined in appended claim 1, and its manufacturing method according to the invention is defined in appended claim 6.

### BRIEF DESCRIPTION OF THE DRAWINGS

The above and/or other aspects and advantages of the present invention will be more apparent and easy to understand from the following description of drawings for the embodiments. Herein:
FIG.1 is an assembly isometric view illustrating an inner structure and an outer structure of a ureteral stent assembly according to an embodiment of the disclosure;
FIG.2 is an assembly top view illustrating an inner structure and an outer structure of a ureteral stent assembly according to an embodiment of the disclosure;
FIG.3 is an isometric view of an outer structure according to an embodiment of the disclosure;
FIG.4 is an enlarged view illustrating the area A of FIG.3;
FIG.5 is an isometric view of another outer structure according to an embodiment of the disclosure;
FIG.6 is an enlarged view illustrating the area B of FIG.5;
FIG.7 is a schematic view of a first inner structure according to an embodiment of the disclosure;
FIG.8 is a schematic view of a second inner structure according to an embodiment of the disclosure;
FIG.9 is an enlarged view illustrating the area C of FIG.8;
FIG.10 is a schematic view of a third inner structure according to an embodiment of the disclosure;
FIG.11 is a schematic view of a fourth inner structure according to an embodiment of the disclosure;

The above drawings use reference signs as follows.

| Reference signs | Parts |
|---|---|
| 100 | outer structure |
| 110 | first wire |
| 200 | inner structure |
| 210 | second wire |

### DETAILED DESCRIPTION OF ILLUSTRATED EMBODIMENTS

The embodiments of the disclosure will be described in detail below. Examples of the embodiments are illustrated in the drawings. It should be understood that identical or same reference signs throughout the text indicate the identical or same elements or the elements with identical or same functions. The embodiments described below in conjunction with the drawings are exemplary and are intended for purposes of illustration only, and are not intended to limit the scope.

It should be understood that, for convenience of description and simplify the explanation, the terms such as "up", "down", "front", "rear", "left" and "right" used in the description refer to position and orientation relationships depicted in the drawings. It is not intended to limit the disclosure by indicating or hinting devices or components having particular orientation relationships or being construed or operated with particular orientation relationships.

In the description of the disclosure, the term "several" means one or plurality, the term "plurality" means two or more than two, terms such as "greater than", "less than" and "exceed" should be understood to exclude the boundary value, and terms such as "above", "below" and "within" should be understood to include the boundary value. In addition, terms such as "first" and "second" used herein are merely intended to distinct the features from each other, but are not intended to indicate or hint relative importance or quantity or precedence relationship of the characteristics.

Unless defined otherwise, terms such as "arrange", "mount" and "connect" used herein are intended to have the meanings commonly understood in a broad sense based on particular content of the technical solution by those skilled in the art.

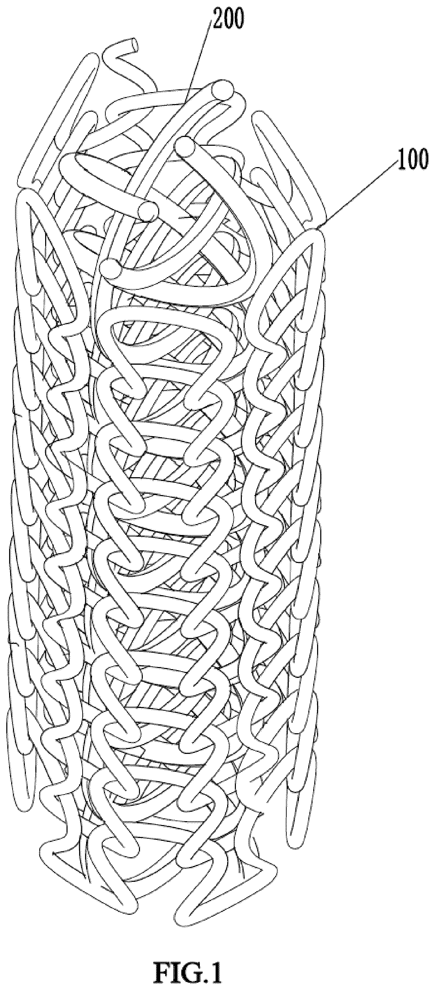

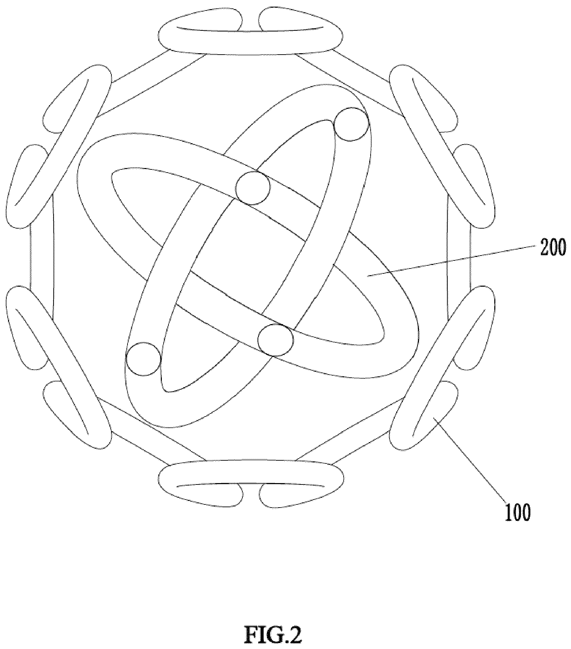

Referring to FIGs.1 and 2, a ureteral stent according to an embodiment of the first aspect of the disclosure comprises an inner structure 200, and an outer structure 100 sleeved on the inner structure 200. Herein, the outer structure 100 is a tube-shaped structure, and the outer structure 100 is knitted with the first wire 110.

The ureteral stent according to the present embodiment of the first aspect of the disclosure can be applied as follows. During use, the tube-shaped outer layer may be radially compressed and then placed in the urethra by an implant device, to provide a support in the urethra. When the outer structure 100 deforms, the outer structure 100 supported by the inner structure 200 would not deform too much to completely fold up due to the inner structure 200 that provides a support inside the outer structure 100. In such a case, the urine can flow along the gaps formed by the first wire 110 of the outer layer. Thus, the outer structure 100 still has an effect on urinary catheterization. Consequently, the ureteral stent failure in fluid guiding can be effectively reduced.

In another aspect, in the case that the outer structure 100 is a structure formed by tightly knitting the first wire 110, fluid guiding gaps for fluid guiding can be formed by the first wire 110. In addition, the tightly knitted structure can prevent proliferative tissues from accessing to the passage and blocking the urine guiding passage.

Herein, in order to improve deformation performance of the ureteral stent to enable the outer structure 100 to provide a support for the urethra even when the outer structure 100 is compressed, it is conceivable to use the first wire 110 which is made of elastic thread. Herein, the elastic thread may be made from hyperelastic materials of shape memory alloy, for example, Nitinol alloy. Alternatively, it is also conceivable to use high polymer materials having high elasticity.

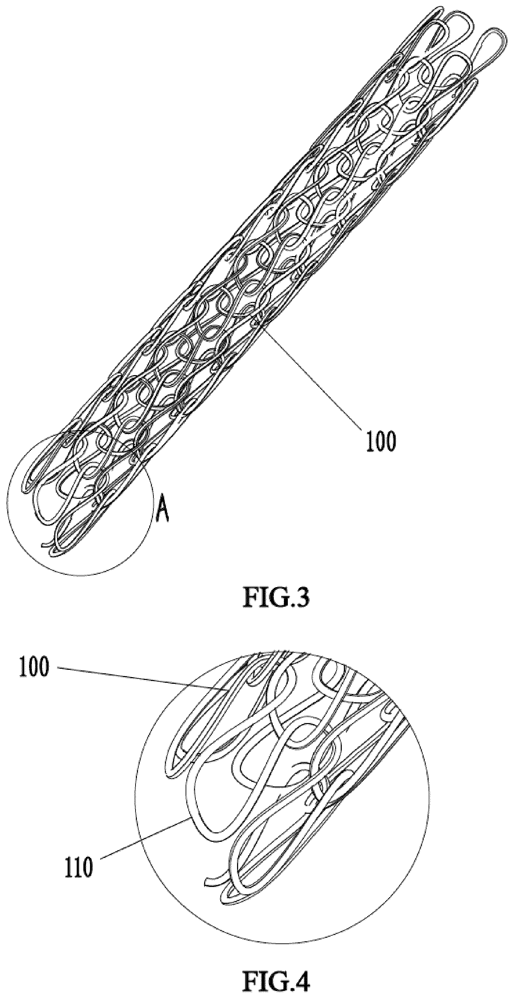

Referring to FIGs.3 and 4, the outer structure 100 may be produced by knitting method. In such a case, the first wire 110 may be bent to form loops passing through one another to constitute the outer structure 100.

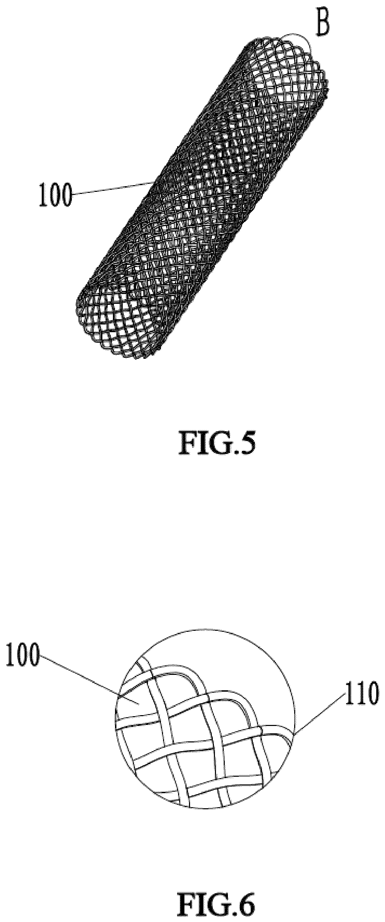

Referring to FIGs.5 and 6, the outer structure 100 may be produced by woven method. In such a case, segments of the first wire 110 interlace with one another in a first direction and a second direction to constitute the outer structure 100. Herein, the first direction and the second direction are perpendicular to each other.

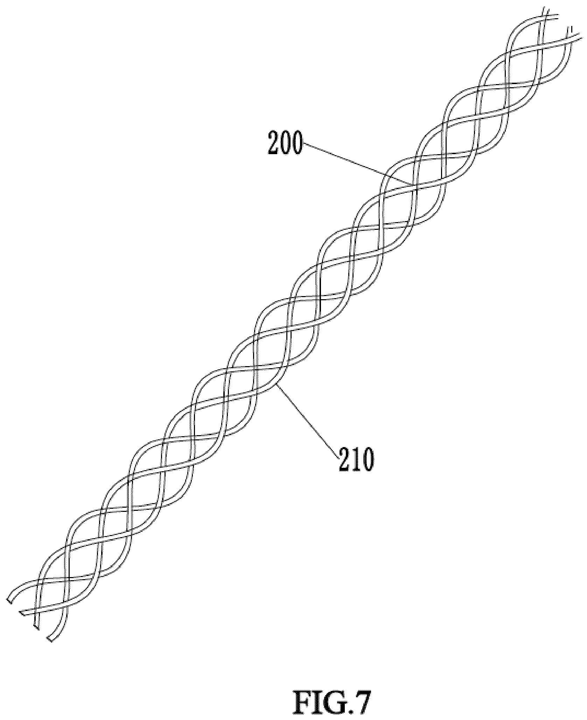

Referring to FIGs.7-11, the inner structure 200 is at least made of a strand of second wire 210, and the second wire 210 that is made is provided with fluid guiding gaps for fluid guiding. Herein, the urine can flow through the fluid guiding gaps formed by the second wire 210 of the inner layer and gradually flow from the renal pelvis to the bladder. In such a case, the outer structure 100 can have a good effect on urine guiding even when being compressed.

In the embodiment, both ends of the ureteral stent are bent, and the two ends of the ureteral stent are bent in opposite directions. In such a case, the ureteral stent may have a profile similar to the pigtail stents widely used in the prior arts for urinary catheterization, which effectively avoids displacement of the ureteral stent in the ureter.

A first aspect of the embodiment further provides a manufacturing method of the ureteral stent for manufacturing the above-mentioned ureteral stent. The method comprises steps as follows: preparing the outer structure 100 with the first wire 110, and then sleeving the outer structure 100 on the inner structure 200.

Herein, the outer structure 100 may be produced by knitting method, in which the first wire 110 is bent to form loops passing through one another to constitute the outer structure 100. Alternatively, it may be produced by woven method, in which segments of the first wire 110 interlacing in the first direction and the second direction constitute the outer structure 100. Herein, the first direction and the second direction are perpendicular to each other. In the woven method, the first wire 110 extending in the first direction during preparing is the warp strand, and the second wire 210 extending in the second direction during preparing is the weft strand.

The second aspect of the embodiment further provides a ureteral stent, which comprises an inner structure 200, and an outer structure 100 sleeved on the inner structure 200. Herein, the inner structure 200 is provided with fluid guiding gaps which allow the urine to flow therethrough.

The ureteral stent according to the present embodiment of the second aspect of the disclosure can be applied as follows. During use, the tube-shaped outer layer may be compressed and then placed in the urethra by an implant device, to provide a urine guiding passage in the urethra. In such a case, the urine can flow along the fluid guiding gaps formed by the inner structure 200 even when the outer structure 100 is maximally compressed to tightly abut against the inner layer. Thus, the ureteral stent failure in fluid guiding is effectively reduced.

Referring to FIGs.7-11, the inner structure 200 is a rope-shaped structure made from the second wire 210, and the second wire 210 that is woven is provided with fluid guiding gaps. As the inner structure 200 is a rope-shaped structure which cannot be radially compressed, it has a good effect on fluid guiding when the outer structure 100 is compressed. In addition, the second wire 210 that is woven provides fluid guiding gaps for urine guiding, such that the ureteral stent can have a good effect on fluid guiding even when being compressed.

Similar to the first wire 110, the second wire 210 may be made of elastic thread. Herein, the elastic thread may be made from hyperelastic materials of shape memory alloy, for example, nickel titanium alloy. Alternatively, it is also conceivable to use high polymer materials having high elasticity.

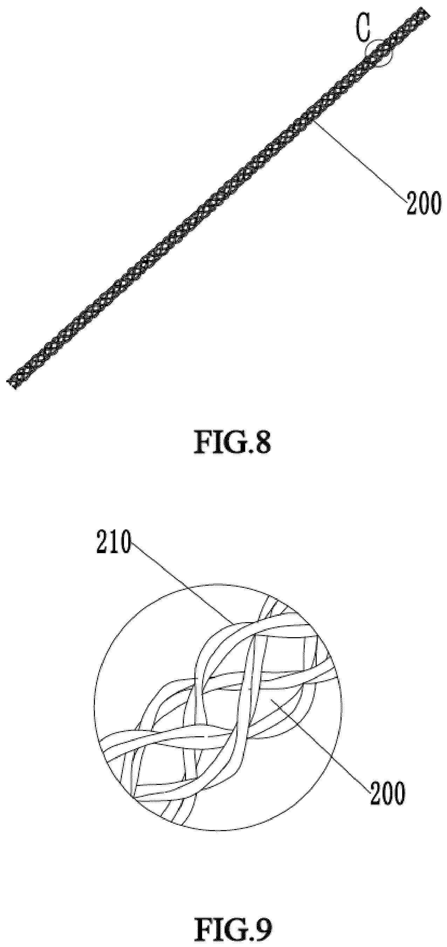

Referring to FIGs.8-11, the second wire 210 may be woven to the inner structure 200 in various manners. Details are discussed below.

Referring to FIG.7, the inner structure 200 is formed by preparing four strands of the second wire 210 interlaced with one another.

Referring to FIGs.8 and 9, the inner structure 200 is formed by preparing four groups of wire strands interlaced with one another, and each one group of wire strands is formed by two threads of the second wire 210 intertwining with each other.

Apparently, it is conceivable that the inner structure 200 may be formed by four strands of the second wire 210 intertwined in a clockwise direction.

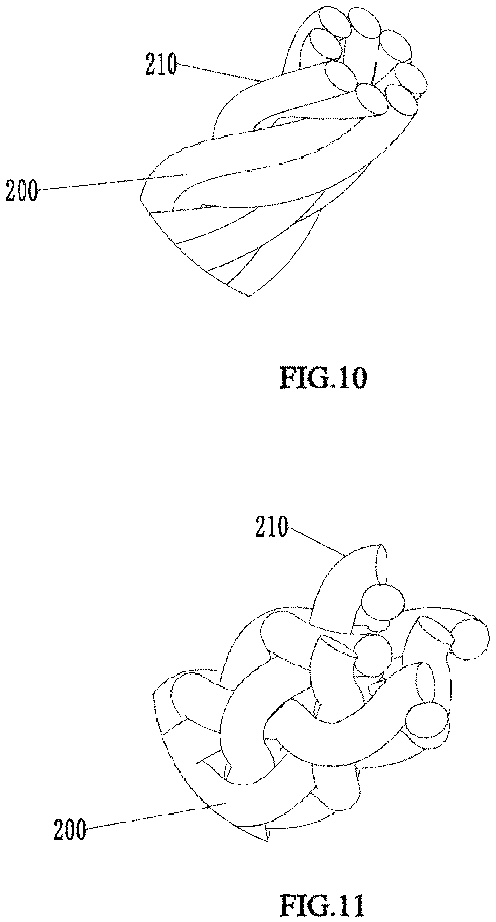

Referring to FIG.10, the inner structure 200 is formed by four groups of wire strands intertwined in a clockwise direction, and each one group of wire strands is formed by two threads of the second wire 210 intertwining with each other.

Referring to FIG.11, the inner structure 200 is formed by eight threads of the second wire 210 intertwining in a first direction, and adjacent two threads of the second wire 210 intertwine in a second direction opposite to the first direction.

In order to improve support function of the ureteral stent, the inner wall of the outer structure 100 can tightly abut against the outer wall of the inner structure 200. In such a case, the inner structure 200 can support the outer structure 100 well. In the case that both inner and outer layers are made from wires, the urine not only can flow in the outer wall gap of the outer structure 100 and the inner wall gap of the inner structure 200, but also can permeate through the gaps between the interlaced wires of the inner and outer layers.

It should be noted that the ureteral stent according to all aspects of the disclosure may be combined in a single application of the ureteral stent with excellent functions.

The description of embodiments described above in detail in conjunction with the drawings is not intended to limit the scope of the invention in any form. The invention is intended to cover all changes made by those skilled in the art without departing from the invention as defined in the following claims.

## Claims

1. A ureteral stent, comprising:
an inner structure (200), and
an outer structure (100) sleeved on the inner structure (200),
wherein the outer structure (100) is a tube-shaped structure that is radially compressible, and the outer structure (100) is knitted with a first wire (110), and
the inner structure (200) is a rope-shaped structure that is radially uncompressible, the inner structure (200) is woven with a second wire (210), and the second wire (210) that is woven defines fluid guiding gaps for fluid guiding therein.

2. The ureteral stent according to claim 1, wherein the first wire (110) is an elastic thread.

3. The ureteral stent according to claim 2, wherein the first wire (110) is a memory alloy thread.

4. The ureteral stent according to claim 1, wherein the first wire (110) is bent to form loops passing through one another to constitute the outer structure (100).

5. The ureteral stent according to claim 1, wherein both ends of the ureteral stent are bent, and the two ends of the ureteral stent are bent in opposite directions.

6. A manufacturing method of a ureteral stent, for manufacturing the ureteral stent according to claim 1, comprising steps of:
knitting the outer structure (100) with the first wire (110),
weaving the inner structure (200) with the second wire (210), and
then sleeving the outer structure (100) on the inner structure (200).

7. The manufacturing method of a ureteral stent according to claim 6, wherein knitting the outer structure (100) with the first wire (110) comprises bending the first wire (110) to form loops passing through one another to constitute the outer structure (100).

## Patentansprüche

1. Ein Harnröhrenstent, umfassend:
eine innere Struktur (200), und
eine äußere Struktur (100), die auf die innere Struktur (200) überzogen ist, wobei
die äußere Struktur (100) eine rohrförmige Struktur ist, die radial komprimierbar ist, und die äußere Struktur (100) mit einem ersten Draht (110) gestrickt ist, und
die innere Struktur (200) eine seilförmige Struktur ist, die radial inkompressibel ist, die innere Struktur (200) mit einem zweiten Draht (210) gewebt ist, und der gewebte zweite Draht (210) flüssigkeitsleitende Spalte zur Flüssigkeitsleitung darin definiert.

2. Der Harnröhrenstent nach Anspruch 1, wobei der erste Draht (110) ein elastischer Faden ist.

3. Der Harnröhrenstent nach Anspruch 2, wobei der erste Draht (110) ein Faden aus einer Formgedächtnislegierung ist.

4. Der Harnröhrenstent nach Anspruch 1, wobei der erste Draht (110) gebogen ist, um Schlaufen zu bilden, die durch einander verlaufen, um die äußere Struktur (100) zu bilden.

5. Der Harnröhrenstent nach Anspruch 1, wobei beide Enden des Harnröhrenstents gebogen sind, und die zwei Enden des Harnröhrenstents in entgegengesetzte Richtungen gebogen sind.

6. Ein Herstellungsverfahren für einen Harnröhrenstent zur Herstellung des Harnröhrenstents nach Anspruch 1, umfassend die Schritte:
stricken der äußeren Struktur (100) mit dem ersten Draht (110),
weben der inneren Struktur (200) mit dem zweiten Draht (210), und
anschließendes überziehen der äußeren Struktur (100) auf die innere Struktur (200).

7. Das Herstellungsverfahren für einen Harnröhrenstent nach Anspruch 6, wobei das Stricken der äußeren Struktur (100) mit dem ersten Draht (110) das Biegen des ersten Drahtes (110) umfasst, um Schlaufen zu bilden, die durch einander verlaufen, um die äußere Struktur (100) zu bilden.

## Revendications

1. Une endoprothèse urétérale, comprenant:
une structure interne (200), et
une structure externe (100) enfilée sur la structure interne (200),
dans laquelle
la structure externe (100) est une structure de forme tubulaire qui est radialement compressible, et la structure externe (100) est tricotée avec un premier fil (110), et
la structure interne (200) est une structure en forme de corde qui est radialement incompressible, la structure interne (200) est tissée avec un second fil (210), et le second fil (210) qui est tissé définit des espaces de guidage de fluide pour le guidage de fluide à l'intérieur de celle-ci.

2. L'endoprothèse urétérale selon la revendication 1, dans laquelle le premier fil (110) est un fil élastique.

3. L'endoprothèse urétérale selon la revendication 2, dans laquelle le premier fil (110) est un fil en alliage à mémoire de forme.

4. L'endoprothèse urétérale selon la revendication 1, dans laquelle le premier fil (110) est plié pour former des boucles passant les unes à travers les autres pour constituer la structure externe (100).

5. L'endoprothèse urétérale selon la revendication 1, dans laquelle les deux extrémités de l'endoprothèse urétérale sont courbées, et les deux extrémités de l'endoprothèse urétérale sont courbées dans des directions opposées.

6. Un procédé de fabrication d'une endoprothèse urétérale, pour fabriquer l'endoprothèse urétérale selon la revendication 1, comprenant les étapes consistant à:
tricoter la structure externe (100) avec le premier fil (110),
tisser la structure interne (200) avec le second fil (210), et
ensuite enfiler la structure externe (100) sur la structure interne (200).

7. Le procédé de fabrication d'une endoprothèse urétérale selon la revendication 6, dans lequel le tricotage de la structure externe (100) avec le premier fil (110) comprend le pliage du premier fil (110) pour former des boucles passant les unes à travers les autres pour constituer la structure externe (100).
